# EUROPEAN PATENT SPECIFICATION

(11) **EP 3 469 554 B1**
(45) Date of publication and mention of the grant of the patent: **09.09.2020**
(21) Application number: 17734995.8
(22) Date of filing: 31.05.2017
(51) Int. Cl.: G06T 15/08, A61B 8/08, G06T 15/50

(54) **SYSTEMS AND METHODS FOR LIGHTING IN RENDERED IMAGES**
SYSTEME UND VERFAHREN ZUR BELEUCHTUNG IN GERENDERTEN BILDERN
SYSTÈMES ET PROCÉDÉS D'ÉCLAIRAGE DANS DES IMAGES RENDUES

(30) Priority: 10.06.2016 US 201662348272 P; 07.11.2016 EP 16306454
(43) Date of publication of application: 17.04.2019
(73) Proprietor: Koninklijke Philips N.V., 5656 AG Eindhoven (NL)
(72) Inventor: MORY, Benoit Jean-Dominique Bertrand Maurice, 5656 AE Eindhoven (NL); ATTIA, Emmanuel Mocé Serge, 5656 AE Eindhoven (NL); ROUET, Jean-Michel, 5656 AE Eindhoven (NL)
(74) Representative: Philips Intellectual Property & Standards
(86) International application number: PCT/EP2017/063080
(87) International publication number: WO 2017/211626

(56) References cited:
- WO-A1-2016/032717

## Description

### BACKGROUND

In medical imaging, images may be rendered in real time or post-data set acquisition. The images may be two dimensional (2D) slices or planes acquired within a volume or the images may be three dimensional (3D) volumes. 3D volume rendering techniques may involve casting virtual rays into an imaged 3D volume to obtain a 2D projection of the data that may be displayed in a final rendered image. The data may include anatomic structures within the imaged volume. When rays are cast from a virtual observer's position towards a region of interest within the imaged volume, various anatomic structures may be interposed along the line of sight. Incoming light direction drives the appearance of shadows and reflections on the surfaces of the anatomic structures. Use of a simulated light source in rendering the image may provide a user with a sense of depth and how the various anatomic structures are arranged in the 3D volume. One or more anatomic structures may block or otherwise interfere with obtaining a clear image of the region of interest. The user may rotate the 3D volume, which may change the position of the virtual observer and/or simulated light source relative to the 3D volume. A new 2D projection of the data may be rendered. Shadows and other lighting effects from the simulated light source may shift based on the rotation of the 3D volume, providing the user with additional information on depth and arrangement of anatomical features.

For a given 3D image data set, image rendering techniques are used to produce a 2D image from a given viewpoint by making assumptions about the optical properties of tissue being imaged under a light source of a predefined color and intensity. Currently, image rendering techniques for ultrasound imaging systems rely on a directional light source located at a fixed distance or infinity. The incoming light direction may be presented to a user by an arrow on a trackball-controlled dedicated sphere widget. In addition to rotating the 3D volume, the user may change the direction of incoming light from the simulated light source.

WO 2016/032717 A1 discloses a method including accessing a 3D medical imaging data set and generating a volume-rendered image from the 3D medical imaging data set. Generating the volume-rendered image includes calculating a shading for the volume-rendered image based on a first light source, a second light source, and a third light source. The second light source and the third light source are both positioned differently than the first light source. Further, the method includes displaying the volume-rendered image.

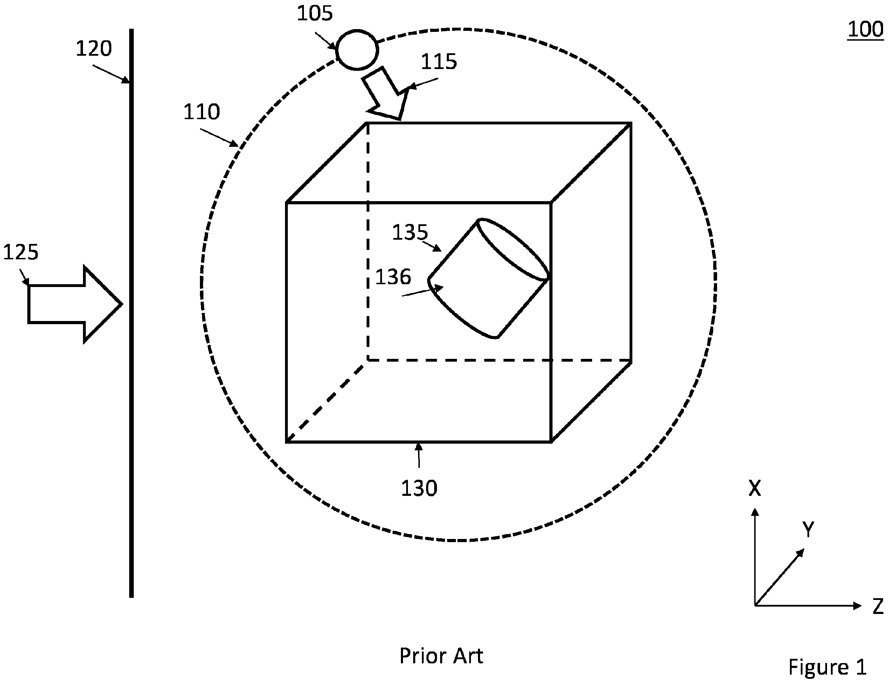

Figure 1 is a schematic illustration of an example of an existing image rendering technique 100. A 3D data set 130 may have been acquired by an ultrasound probe or other imaging technique. The 3D data set 130 may include data corresponding to a 3D volume in a body. The 3D data set 130 may include a region of interest 135. The region of interest 135 may be a portion of an object (e.g., wall of blood vessel, valve of heart) or may be an entire object (e.g., tumor, fetus). When rendering an image of the 3D data set 130 including the region of interest 135, a simulated light source may be used to provide shadows and reflections on one or more surfaces within the 3D data set 130, for example, a surface 136 of the region of interest 135, which may provide depth perception for a user. The simulated light source may be a directional light source 105. The directional light source 105 may transmit light only in a direction indicated by arrow 115. The user may be permitted to select a position of the directional light source 105 at a fixed distance 110 from the 3D data set 130. A 2D projection of the 3D data set 130 may be rendered relative to display image plane 120, based on a virtual observer observing the 3D data set 130 from a viewpoint indicated by arrow 125. Display image plane 120 may be aligned with the X-Y plane of the 3D data set 130. Arrow 125 may be perpendicular to image plane 120. That is, a virtual observer may be considered to be "looking" through the image plane 120 at the 3D data set 130 through the depth of the 3D data set 130 indicated by the Z-axis. The 2D projection at display image plane 120 of the 3D data set 130 may be provided as an image to a user on a display.

Although the user may move the directional light source 105 about the 3D data set 130, locating the directional light source 105 outside of a rendered volume may cause object self-shadowing and make it difficult to illuminate structures of the region of interest 135. Details of the volume and/or region of interest 135 may be obscured. Anatomic details inside concave cavities may not be visible without cropping of the 3D data set 130 or other significant adjustments.

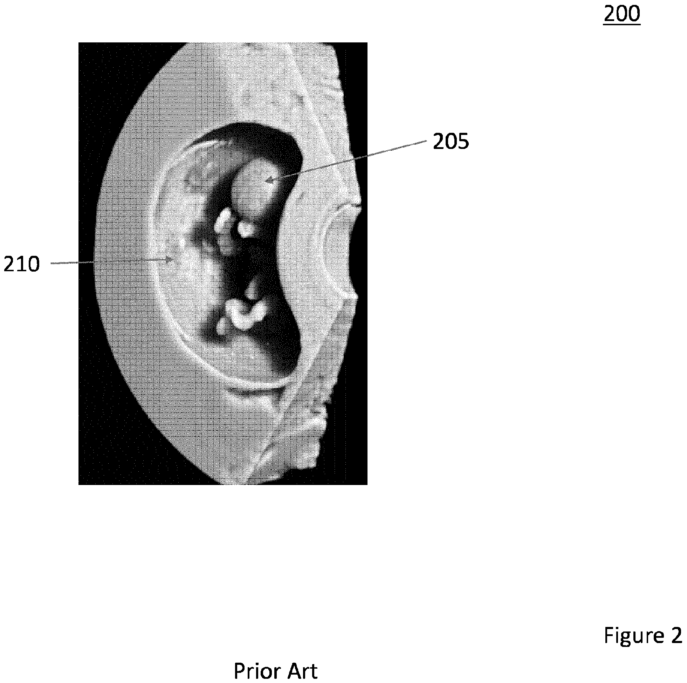

Figure 2 is an example of an image 200 rendered from a 3D data set using an external directional light source. The image 200 displays a fetus 205 within a uterus 210. Many anatomical structures of the fetus 205 are obscured by shadows cast by the uterus 210 based on an image rendering technique using a directional light source located outside the uterus 210. This may inhibit the user, which may be a sonographer, obstetrician, or other clinician, from making a diagnosis or being able to navigate within the volume defined by the 3D data set.

### SUMMARY

An ultrasound imaging system according to the present invention includes the features of claim 1. At least one embodiment of the disclosure may include an ultrasound probe that may be configured to receive ultrasound echoes from a subject to image a volume of the subject, a scan converter that may be configured generate a three dimensional (3D) data set from the ultrasound echoes, a volume renderer that may be configured to calculate surface shading information of a surface of the 3D data set based, at least in part, on a location of a simulated light source relative to the 3D data set and render a two dimensional (2D) projection image of the 3D data set, the 2D projection image including the shading information, and a user interface which may include a display that may be configured to display the 2D projection image, and an input device that may include a user input element that may be configured to receive user input to position the simulated light source at a location behind the surface of the 3D data set. In some embodiments, the simulated light source may be a multidirectional light source.

A method according to the present invention includes the features of claim 11. At least one embodiment of the disclosure may include receiving a selection of a simulated light source for rendering a 2D projection image of a 3D data set, wherein the 3D data set may be constructed from ultrasound echoes received from a volume of a subject, receiving an indication, responsive to user input, of an in-plane position of the simulated light source in a plane corresponding to a projection plane of the 2D projection image, determining a depth position of the simulated light source on an axis normal to the projection plane, calculating surface shading information of a surface of the 3D data set based, at least in part, on the in-plane and depth positions, and rendering the 2D projection image including the shading information on a display.

### BRIEF DESCRIPTION OF THE DRAWINGS

FIG. 1 is a schematic illustration of an image rendering technique using an external directional light source.
FIG. 2 is an example of a rendered image using the image rendering technique shown in FIG. 1.
FIG. 3 is a block diagram of an imaging system according to embodiments of the present disclosure.
FIG. 4 is a schematic illustration of an image rendering technique using a simulated light source according to an embodiment of the present disclosure.
FIG. 5 is an example of a rendered image using the image rendering technique shown in FIG. 4.
FIG. 6 is a schematic illustration of the image rendering technique shown in FIG. 4.
FIGS. 7A-C are examples of rendered images using the image rendering technique shown in FIG. 6.
FIG. 8 is an illustration of a user interface according to an embodiment of the disclosure.
FIG. 9 is a schematic illustration of a display of a user interface according to an embodiment of the disclosure.
FIG. 10 is a schematic illustration of a display of a user interface according to an embodiment of the disclosure.
FIGS. 11A-C are examples of rendered images according to an embodiment of the disclosure.
FIG. 12 is a flowchart of a method according to an embodiment of the disclosure.

### DETAILED DESCRIPTION

The following description of certain exemplary embodiments is merely exemplary in nature and is in no way intended to limit the invention or its applications or uses. In the following detailed description of embodiments of the present systems and methods, reference is made to the accompanying drawings which form a part hereof, and in which are shown by way of illustration specific embodiments in which the described systems and methods may be practiced. These embodiments are described in sufficient detail to enable those skilled in the art to practice the presently disclosed systems and methods, and it is to be understood that other embodiments may be utilized and that structural and logical changes may be made without departing from the spirit and scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

In some applications, it may be desirable to render an image from a 3D data set using a simulated light source positioned within the 3D data set. In some applications, it may be desirable to render an image from a 3D data set using a simulated light source within a region of interest of the 3D data set. In some applications, it may be desirable for the simulated light source to be a multidirectional light source. For example, the simulated light source may be modeled as a sphere that projects light from the entire surface of the sphere in all directions. In another example, the simulated light source may be modeled as a point source that projects light in all directions. Allowing a user to place the simulated light source within the 3D data set may provide rendered images that are less obscured by shadows and/or other artifacts that are generated when an image is rendered with a simulated directional light source located outside the 3D data set. Compared to lighting with an external light source, the close-range lighting may provide better local depth perception of shape and curvature of objects. An image rendered with a simulated light source within the 3D data set may provide an image that is easier for a clinician or other user to interpret. This may improve the ability of the clinician or other user to make a diagnosis and/or navigate within the 3D data set.

In an illustrative example, a clinician may conduct an ultrasound exam on a patient and acquire a 3D data set from the patient (e.g., a fetus in utero). The imaging system may render an image of a 2D projection of the 3D data set with a simulated multidirectional light source. The clinician may move the light source within the 3D data set, and the imaging system may adjust the rendered image based in part on the new position of the light source. For example, the clinician may touch a touch screen displaying the rendered image along with a visual indicator of the light source (e.g., orb, square, X, etc.) and "drag" the light source to different positions within the image. The clinician may move the light source to investigate different areas of interest. Continuing with this example, the clinician may move the light source to highlight contours of the face of the fetus to check for a cleft pallet. The clinician may then move the light source to illuminate the spine to check for deformities. The clinician may choose to control the location of the light source in the image plane (e.g., an in-plane position, X-Y plane position) as well as the depth of the light source in the 3D data set (Z-axis). The clinician may control the light source during the ultrasound exam or during review of stored images after an exam.

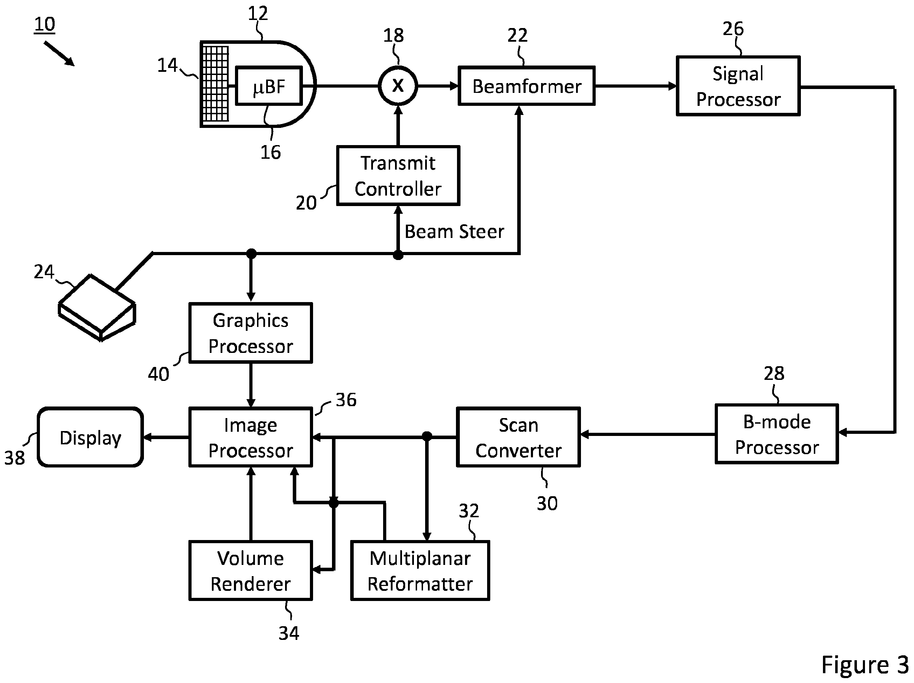

Figure 3 shows a block diagram of an ultrasound imaging system 10 constructed in accordance with the principles of the present disclosure. Although an ultrasound imaging system is shown in explanatory examples of embodiments of the invention, embodiments of the invention may be practiced with other medical imaging modalities. Other modalities may include, but are not limited to, magnetic resonance imaging and computed tomography. The ultrasound imaging system 10 in Figure 3 includes an ultrasound probe 12 which includes a transducer array 14 for transmitting ultrasonic waves and receiving echo information. A variety of transducer arrays are well known in the art, e.g., linear arrays, convex arrays or phased arrays. The transducer array 14, for example, can include a two dimensional array (as shown) of transducer elements capable of scanning in both elevation and azimuth dimensions for 2D and/or 3D imaging. The transducer array 14 is coupled to a microbeamformer 16 in the ultrasound probe 12 which controls transmission and reception of signals by the transducer elements in the array. In this example, the microbeamformer 16 is coupled by the probe cable to a transmit/receive (T/R) switch 18, which switches between transmission and reception and protects the main beamformer 22 from high energy transmit signals. In some embodiments, for example in portable ultrasound systems, the T/R switch 18 and other elements in the system can be included in the ultrasound probe rather than in a separate ultrasound system base. The transmission of ultrasonic beams from the transducer array 14 under control of the microbeamformer 16 is directed by the transmit controller 20 coupled to the T/R switch 18 and the beamformer 22, which receive input from the user's operation of the user interface or control panel 24. One of the functions controlled by the transmit controller 20 is the direction in which beams are steered. Beams may be steered straight ahead from (orthogonal to) the transducer array, or at different angles for a wider field of view. The partially beamformed signals produced by the microbeamformer 16 are coupled to a main beamformer 22 where partially beamformed signals from individual patches of transducer elements are combined into a fully beamformed signal.

The beamformed signals are coupled to a signal processor 26. The signal processor 26 can process the received echo signals in various ways, such as bandpass filtering, decimation, I and Q component separation, and harmonic signal separation. The signal processor 26 may also perform additional signal enhancement such as speckle reduction, signal compounding, and noise elimination. The processed signals are coupled to a B-mode processor 28, which can employ amplitude detection for the imaging of structures in the body. The signals produced by the B-mode processor 28 are coupled to a scan converter 30 and a multiplanar reformatter 32. The scan converter 30 arranges the echo signals in the spatial relationship from which they were received in a desired image format. For instance, the scan converter 30 may arrange the echo signal into a two dimensional (2D) sector-shaped format, or a pyramidal three dimensional (3D) image. In some embodiments, the scan converter 30 may generate a 3D data set from the echo signal. The multiplanar reformatter 32 can convert echoes which are received from points in a common plane in a volumetric region of the body into an ultrasonic image of that plane, as described in U.S. Pat. No. 6,443,896 (Detmer). A volume renderer 34 converts the echo signals of a 3D data set into a projected 3D image as viewed from a given reference point, e.g., as described in U.S. Pat. No. 6,530,885 (Entrekin et al.). In some embodiments, the volume renderer 34 may receive input from the user interface 24. The input may include the given reference point (e.g., viewpoint of a virtual observer), location of a simulated light source, and/or properties of the simulated light source for the rendered projected image. In some embodiments, the volume renderer 34 may calculate surface shading information for one or more surfaces in the 3D data set based at least in part, on the location and/or properties of the simulated light source. The 2D or 3D images are coupled from the scan converter 30, multiplanar reformatter 32, and volume renderer 34 to an image processor 36 for further enhancement, buffering and temporary storage for display on an image display 38. The image processor 36 may render visual cues for the simulated light source (e.g., orb, halo) in some embodiments. In some embodiments, the visual cues may be rendered by the volume renderer 34. The graphics processor 40 can generate graphic overlays for display with the ultrasound images. These graphic overlays can contain, e.g., standard identifying information such as patient name, date and time of the image, imaging parameters, and the like. For these purposes the graphics processor receives input from the user interface 24, such as a typed patient name. The user interface can also be coupled to the multiplanar reformatter 32 for selection and control of a display of multiple multiplanar reformatted (MPR) images.

According to an embodiment of the disclosure, the ultrasound probe 12 may be configured to receive ultrasound echoes from a subject to image a volume of the subject. The scan converter 30 may receive the ultrasound echoes and generate a 3D data set. As described above, the ultrasound echoes may be pre-processed by the beamformer 22, signal processor 26, and/or B-mode processor prior to being received by the scan converter 30. The 3D data set may include values for each point (e.g., voxel) in the imaged volume. The values may correspond to echo intensity, tissue density, flow rate, and/or material composition. Based on the values in the 3D data set, the scan converter 30 and/or volume renderer 34 may define one or more surfaces within the imaged volume. The surfaces may represent a boundary between two different objects (e.g., fetus and uterus) or materials (e.g., bone and muscle), or regions (e.g., different flow rates in a vessel) within the imaged volume. In some embodiments, the surfaces may be an isosurface.

When rendering a 2D projection image of the 3D data set, the volume renderer 34 may receive a location of a simulated light source relative to the 3D data set. In some embodiments, the location of the simulated light source may be pre-programmed by the imaging system 10. The simulated light source may default to a pre-programmed location, e.g., upon activation of a volume rendering mode, and in some cases the light source may be movable by the user while in the volume rendering mode. In some embodiments, the location of the simulated light source may be received via user interface 24, which may include input devices having one or more input elements configured to receive user input. For example, the user interface 24 may include a touch screen with a graphical user interface (GUI) that allows a user to set a location of the simulated light source anywhere within and/or proximate to the 3D data set. As an example, the graphical user interface (GUI) may provide one or more GUI elements that enable the user to set the location of the simulated light source. In some examples, a GUI element (e.g., a light orb) may additionally provide a visual cue as to the location of the light source in relation to the volume. In other examples, the GUI element may be an input widget whereby the user may be able to specify the location (e.g., specify X, Y, Z coordinates) of the light source. Other examples of GUI elements may be used. In yet further examples, the user input may be received via a mechanical control (e.g., a trackball or a rotary encoder on a control panel) which in the volume rendering mode may be specifically associated with and configured to generate manipulation commands for moving the light source.

The volume renderer 34 may calculate surface shading information for one or more surfaces within the 3D data set, based, at least in part, on the location of the simulated light source relative to the 3D data set. The surface shading information may include information regarding the brightness of any given pixel representing a surface of the 3D data set in a rendered 2D projection image, which information may provide three-dimensionality to the otherwise 2D rendered image. In addition to the location of the light source relative to the surface, the surface shading information may be based on properties of the volume adjacent to the surface (e.g., the value of voxels interposed between the light source and the surface). For example, when calculating the shading information for a given surface, the volume renderer 34 may take into account the density of tissue interposed between the simulated light source and the rendered outer surface. When the simulated light source is located in front of a surface of the imaged volume, only zero-value voxels may be interposed between the light source and the surface and an illuminated region on the surface may have a high luminosity or brightness than in instances in which the simulated light source is behind the surface and thus spaced from the surface by non-zero value voxels. Light transmittance through the zero-value voxels of the regions surrounding the rendered 3D data set may be approximated, by known light simulation techniques, to be similar to light transmittance through air, thus light transmittance through non-zero value voxels may be reduced to approximate transmittance through tissue which is denser than air. Thus, when the simulated light source is located behind a surface enclosing a volume of the 3D data set having a density higher than a surrounding volume, the surface shading information calculated by the volume renderer 34 may be different than when the simulated light source is located in front of the surface. For example, the surface shading information may include fewer reflections and appear to "glow" from within when the simulated light source is located behind the surface while the surface shading information may be such that the surface appears more opaque when the simulated light source is located in front of the surface. As will be appreciated, density and other properties of an object positioned in front of a light source will affect the light transmittance through the object, thus the volume renderer 34 is configured to account for the density of material disposed between the light source and the surface being rendered.

Although reference is made to surface shading, the volume renderer 34 may or may not explicitly extract surfaces from the 3D data set for calculating surface shading information. For example, the volume renderer 34 may calculate shading information for every voxel within the 3D data set (e.g., volumetric shading). As previously mentioned, the shading information for each voxel may be based at least in part on the distance of the voxel from the simulated light source, the density of the voxel, and/or density of surrounding voxels. The resulting shading information for the 3D data set may provide the appearance of 3D surfaces within the 3D data set to a user. For simplicity, the shading information of surfaces of objects and/or areas of interest within the 3D data set will be referred to as surface shading information without regard to the manner in which it is calculated by the volume renderer 34.

The surface shading information may be used by the volume renderer 34 to render the 2D projection image. The rendered 2D projection image may be provided by the volume renderer 34 to the image processor 36 in some embodiments. The rendered 2D projection image may be provided to the display 38 for viewing by a user such as a clinician. In some examples, the rendering by the volume renderer 34 and the resulting 2D projection image provided on the display 38 may be updated responsive to user inputs via the user interface 24, for example to indicate movement (e.g., translation or rotation) of the volume, movement of the simulated light source in relation to the volume, and/or other changes to parameters associated with the various rendering constructs in the rendering.

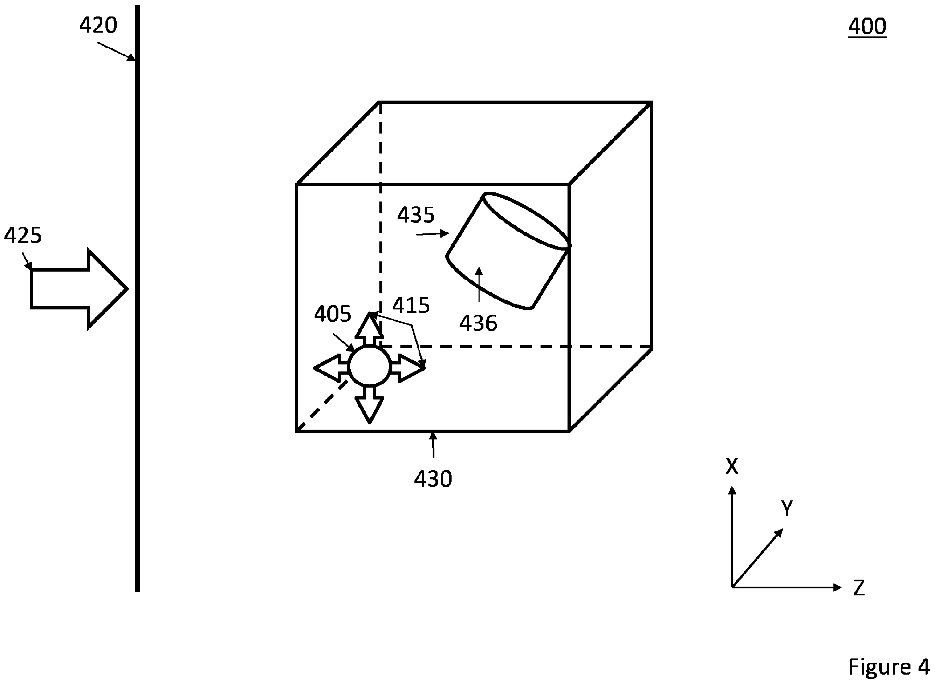

Figure 4 is a schematic illustration of an image rendering technique 400 according to an embodiment of the disclosure. In some embodiments, the image rendering technique 400 may be performed by an imaging system such as ultrasound imaging system 10. A 3D data set 430 may have been acquired by an ultrasound imaging system, such as the ultrasound imaging system 10 shown in Figure 3, or another imaging system (e.g., a magnetic resonance imaging (MRI) machine). The 3D data set 430 may include data corresponding to a 3D volume in a body. The 3D data set 430 may include a region of interest 435. The region of interest 435 may be a portion of an object (e.g., wall of blood vessel, valve of heart) or may be an entire object (e.g., tumor, fetus). In some embodiments, the 3D data set 430 may include multiple regions of interest 435. A 2D projection image of the 3D data set 430 may be rendered relative to display image plane 420, based on a virtual observer observing the 3D data set 430 from a viewpoint indicated by arrow 425. Display image plane 420 may be aligned with an X-Y plane. The vector indicated by arrow 425 may pass through image plane 420. That is, a virtual observer may be considered to be "looking" through the image plane 420 at the 3D data set 430 through the depth of the 3D data set 430 indicated by the Z-axis, which is orthogonal to the X-Y plane. Although shown perpendicular to image plane 420, arrow 425 may be at some other angle relative to image plane 420 (e.g., 10, 30, 45 degrees). The 2D projection image at display image plane 420 of the 3D data set 430 may be provided as an image to a user on a display, such as display 38 shown in Figure 3 When rendering an image of the 3D data set 430 including the region of interest 435, a simulated light source 405 may be used to calculate surface shading information to render shadows and reflections on one or more surfaces within the 3D data set 430, for example, a surface 436 of the region of interest 435, which may provide depth perception for a user. The surface shading information may be based, at least in part, on the position of the simulated light source 405 relative to the 3D data set 430 and/or region of interest 435. In some embodiments, the simulated light source 405 may be a multidirectional light source. The light source 405 may transmit light in all directions as indicated by arrows 415. Unlike the light source 105 shown in Figure 1, the user may be permitted to select a position of the light source 405 outside of or anywhere within the 3D data set 430. As shown in the embodiment illustrated in Figure 4, the light source 405 is within the 3D data set 430 at a depth less than a depth of the region of interest 435. That is, the light source 405 is at a depth along the Z-axis between the region of interest 435 and the virtual observer looking from a direction indicated by arrow 425.

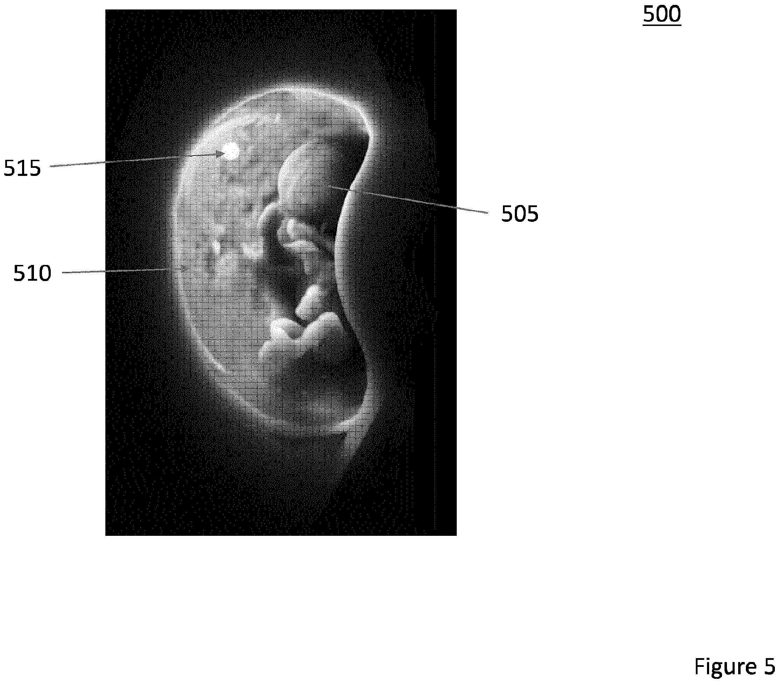

Figure 5 is an example image 500 rendered using the image rendering technique 400 shown in Figure 4. The image 500 is rendered from the same 3D data set as image 200 shown in Figure 2, a fetus 505 within a uterus 510. In some embodiments, the simulated light source may be rendered as an emissive material in the image. In the example shown in image 500, the simulated light source is rendered as a glowing orb 515. The glowing orb 515 is rendered within the 3D data set within the uterus 510. As a result, the uterus 510 does not cast shadows that obscure the fetus 505. In contrast with the fetus 205 in Figure 2, the left arm, right shoulder, and torso of fetus 505 may be discerned. These same features are obscured by uterine shadows in the image 200 shown in Figure 2.

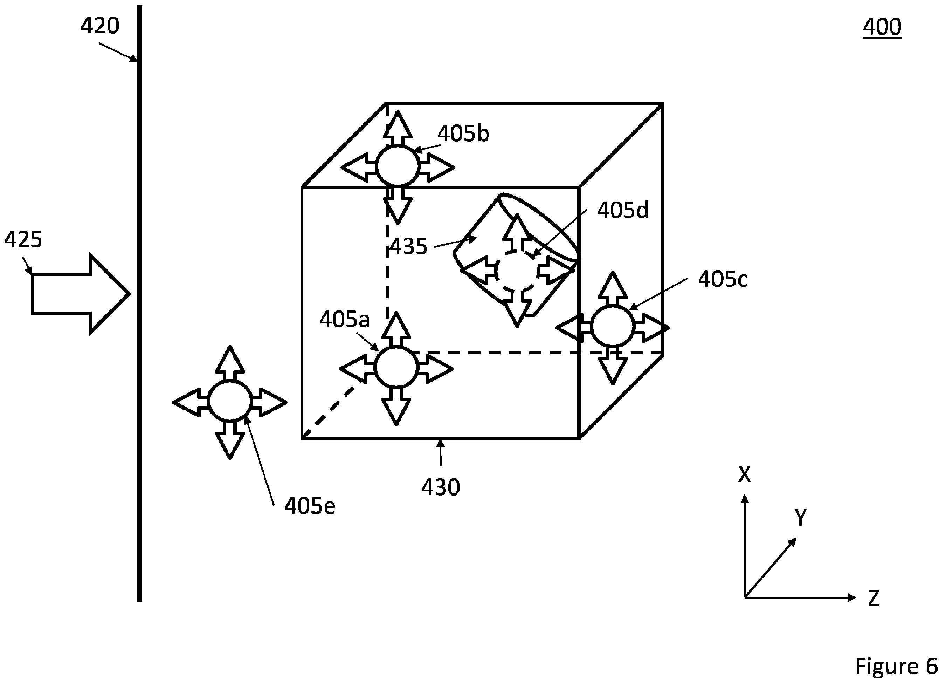

As mentioned previously, the light source 405 is not limited to a set distance from the 3D data set 430. Figure 6 is a schematic illustration of a variety of example possible positions of the light source 405a-e according to embodiments of the disclosure. As shown in Figure 6, the light source 405 may be rendered at varying positions in the image plane 420 (e.g., different positions on the X-Y plane) and at different depths within the 3D data set 430 (e.g., along the Z-axis). For example, the light source 405a is in the position shown in Figure 4, and light source 405b is at the same depth as light source 405a, but at a different point in image plane 420 in the 3D data set 430. Light source 405c is at both a different point on the image plane 420 and at a different depth in the 3D data set 430. As shown in Figure 6, light source 405c is at a deeper depth than the region of interest 435 with reference to the image plane 420. The light source 405 may even be placed within the region of interest 435, as shown by light source 405d. Light source 405e shows an example of a position outside the 3D data set 430. The example positions are shown for explanatory purposes only.

Although not shown in Figure 6, the simulated light source 405 may be a directional light source rather than a multidirectional light source. In some embodiments, a user may be able to toggle between multidirectional and directional modes. A directional light source within the 3D data set 430 may be desirable in some applications. For example, a user may want to highlight a particular area within the 3D data set while minimizing the illumination to other areas, which may reduce distractions (e.g., a "spotlight" effect).

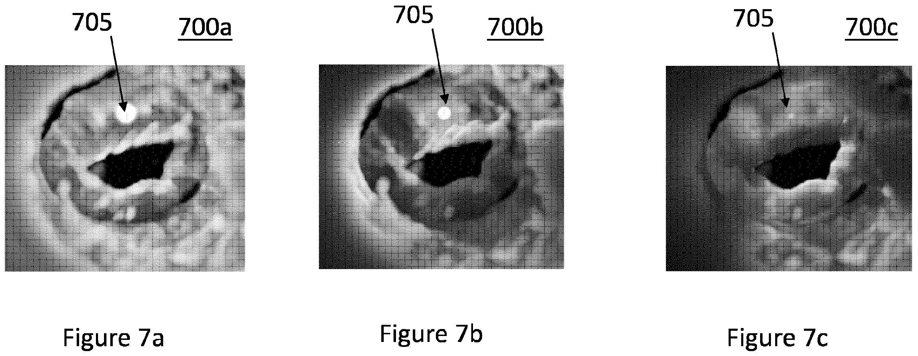

Figures 7a-c are example mitral valve images 700a-c rendered with a simulated light source at different depths according to an embodiment of the disclosure. As shown in Figure 7a, the simulated light source 705 is rendered "in front of" the mitral valve in the image 700a from the perspective of the viewer. Rendering the light source 705 in front of the mitral valve may allow a clinician to discern features on the surface of the mitral valve and the tissue of the heart surrounding the valve. In Figure 7b, the simulated light source 705 is rendered within the mitral valve in image 700b. When the light source 705 is rendered within the mitral valve, the clinician may be able to observe more subtle contours and depths of different components of the mitral valve. Figure 7c is an example of a rendered image with light source 705 positioned behind the mitral valve. Placing the light source behind the mitral valve may be advantageous for at least a qualitative determination of the thickness of the mitral valve in different portions. A clinician may have other reasons and/or there may be additional advantages to the different positions of the light source 705. For example, a clinician may position the light source 705 at a depth to avoid casting shadows from other anatomy on the region of interest.

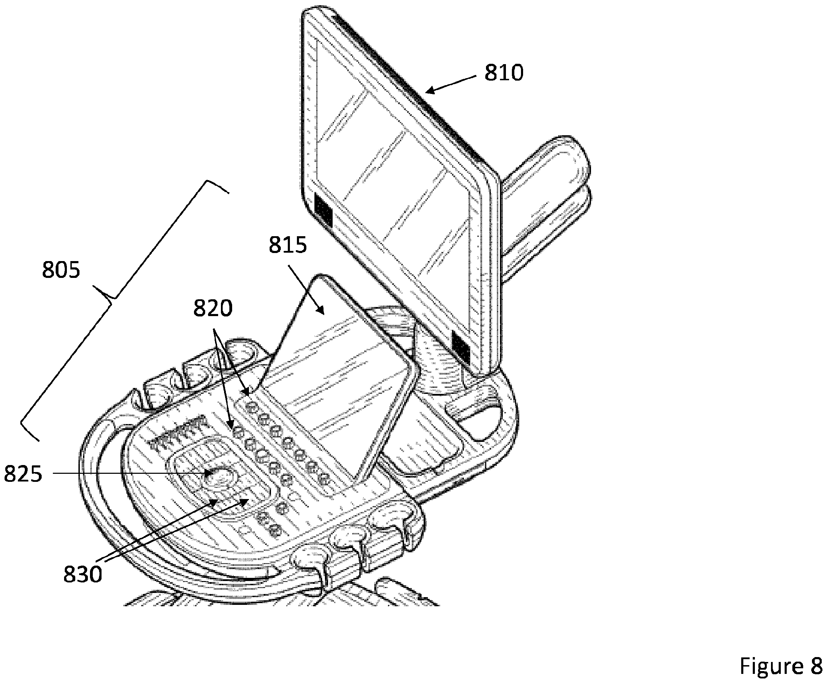

Figure 8 is an illustration of a portion of an ultrasound system 800 that may be used to implement an embodiment of the disclosure. The ultrasound system 800 may include a user interface 805 and a display 810. In some embodiments, user interface 805 may be used to implement user interface 24 shown in Figure 3. The display 810 may be used to implement display 38 shown in Figure 3 in some embodiments. The user interface 805 may include one or more input devices including one or more user input elements. For example, user interface 805 may include a touch screen 815, one or more rotary controls 820, a track ball 825, and buttons 830. In some embodiments, the buttons 830 may include arrow keys and/or a QWERTY keyboard. In some embodiments, the display 810 may also be part of the user interface 805. For example, the display 810 may be implemented using a touch screen. A user may have the option of using the display 810, the touch screen 815, and/or other controls included in the user interface 805 to position the simulated light source in a rendered image and/or control other properties of the simulated light source (e.g., directional vs. multidirectional, intensity, color).

A user may control the position of the simulated light source in a rendered image via a user interface such as the user interface 805 shown in Figure 8. In some embodiments, the user may use the track 825 ball and the rotary control 820. The user may select an in-plane position (e.g., an X-Y coordinate) on the image plane with the track ball 825 and select a depth position (e.g., a coordinate on the Z-axis) with the rotary control 820 to set the position of the simulated light source. In some embodiments, an individual rotary control may be provided for each degree of freedom (e.g., an X-axis control, a Y-axis control, and a Z-axis control) to set the position of the simulated light source. In some embodiments, the user may use buttons 830, such as arrow keys, to select a position (e.g., X-Y-Z coordinate) of the simulated light source.

In some embodiments, the user interface 805 or an input element of the user interface includes a graphical user interface (GUI). For example, the display 810 and/or touch screen 815 may include a GUI. In some embodiments, the user may use the touch screen 815 to position the simulated light source. A variety of gestures on the touch screen 815 may be used to select a position of the simulated light source. For example, the user may tap the touch screen 815 at a location to set the in-plane position and/or touch a rendered light orb in the image displayed on the touch screen 815 and "drag" it to a location by moving their finger along the touch screen 815. Each point on the touch screen 815 may coincide with each point of the image plane. The user may press and hold the touch screen 815 to set the depth position of the light source and/or use "pinch" and "expand" gestures with two or more fingers. In other words, a user may place two fingers on the touch screen 815 close together and slide them apart along the touch screen 815 to increase the depth of the light source within the 3D data set in relation to the image plane. To decrease the depth, the user may place two fingers apart on the touch screen 815 and draw them together. These gestures are provided only as examples, and other gestures may be used to set the position of the simulated light source in the 3D data set (e.g., control buttons provided on touch screen). In some embodiments, a user may position the simulated light source using one or a combination of user input methods. For example, a user may set a position of the simulated light source using the touch screen and then "fine tune" the position using the track ball and/or rotary control. In some embodiments, the user interface 805 may include additional and/or alternative user input controls (e.g., slide control, motion sensor, stylus) for positioning the simulated light source. In some embodiments, the user may use the user interface 810 to control properties of the simulated light source. For example, a user may set an intensity and/or color of the light source.

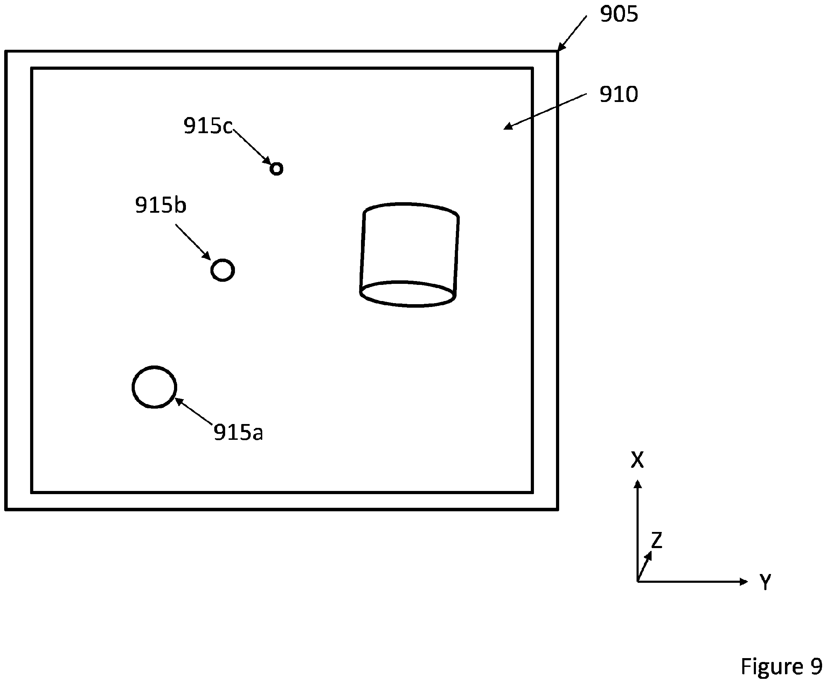

Figure 9 is an illustration of a rendered image 910 on a display 905 according to an embodiment of the disclosure. Display 38 of Figure 3 or display 810 of Figure 8 may be used to implement display 905 in some embodiments. In some embodiments, the display 905 may include a GUI and the simulated light source 915 may be rendered with visual cues to assist a user in positioning the light source. As shown in Figure 9, the simulated light source 915 may be rendered in the image 910 as smaller in size as the light source is positioned farther away from the image plane in the 3D data set. In some embodiments, the image plane aligns with the display 905. As shown in Figure 9, the light source 915 would appear to be moving further into the page. In this example, light source 915a is closest to the image plane and light source 915c is furthest from the image plane. Changing the size of the light source 915 in the image 910 may provide a visual cue indicating a depth of the light source 915 along the Z-axis in the 3D data set and may assist a user in positioning the light source within the 3D data set.

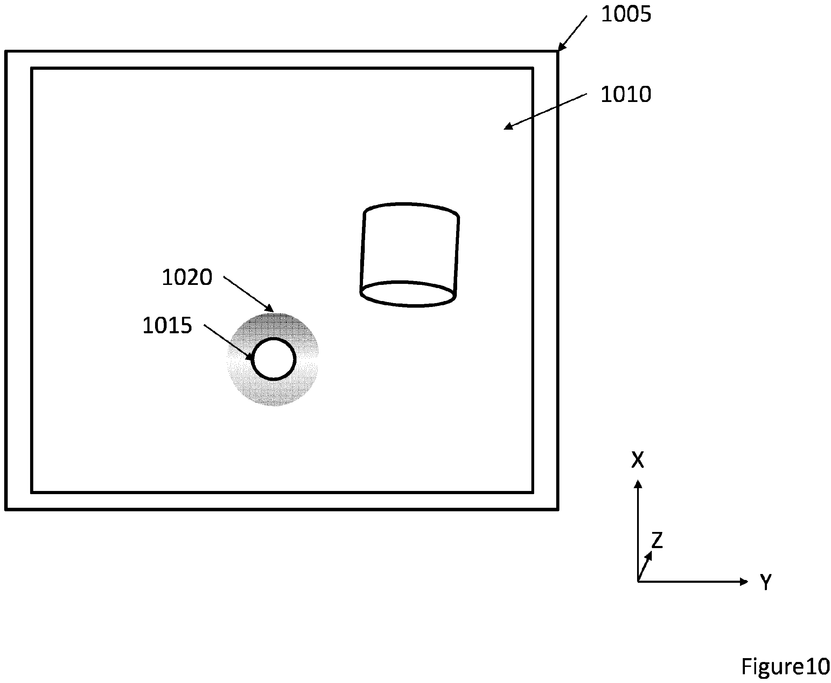

Figure 10 is an illustration of a rendered image 1010 on a display 1005 according to an embodiment of the disclosure. Display 38 of Figure 3 or display 810 of Figure 8 may be used to implement display 1005 in some embodiments. In some embodiments, the display 1005 may include a GUI and the simulated light source 1015 may be rendered in the image 1010 with a halo 1020. The halo 1020 may allow a user to visually locate the light source 1015 in the image 1010. In some embodiments, the halo 1020 may allow the user to locate the light source 1015 when the light source 1015 is positioned outside the field of view of the image 1010. In some embodiments, a user may turn the halo 1020 on and off. That is, the user may control whether or not the halo 1020 is rendered around the light source 1015 in the image 1010. In some embodiments, the halo 1020 may automatically disappear after the light source 1015 has been stationary for a period of time (e.g., half a second, two seconds, ten seconds). In some embodiments, the user may turn off the visual cue of the light source 1015. By turn off, it is not meant that the user chooses to remove the lighting rendered from the light source 1015 from the image 1010, but that the user turns off the rendering of the visual cue of the light source 1015 in the image 1010 (e.g., the orb). In some embodiments, the rendering of the visual cue of the light source 1015 may automatically disappear after the light source 1015 has been stationary for a period of time (e.g., half a second, two seconds, ten seconds). Turning on and off the halo 1020 and/or rendering of the light source 1015 may allow for the user to observe the image 1010 without interference from the visual cues for positioning the light source 1015. Visual cues such as the orb and/or halo may be rendered by a volume renderer and/or image processor of an imaging system. For example, volume renderer 34 and image processor 36 of ultrasound imaging system 10 shown in Figure 1 may be used to implement an embodiment of the disclosure.

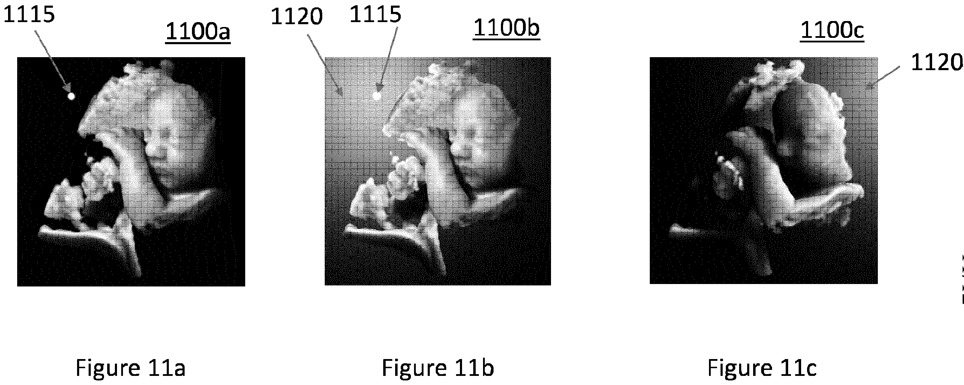

Figure 11a-b are example images of rendered images 1100a-c of alight source 1115 with a halo 1120. Figure 11a shows an image 1100a with a simulated light source 1115 rendered as an orb. Figure 11b shows the light source 1115 rendered with a halo 1120. Some users may find the light source 1115 easier to locate in Figure 11b compared to Figure 11a. The halo 1120 may indicate to a user that the light source 1115 is multidirectional rather than directional. Figure 11c shows image 1100c where the light source 1115 has been positioned outside the user's field of view. However, the user may still locate the light source 1115 because the halo 1120 is within the user's field of view. The halo 1120 may make it easier for a user to locate and position the light source 1115 in some embodiments.

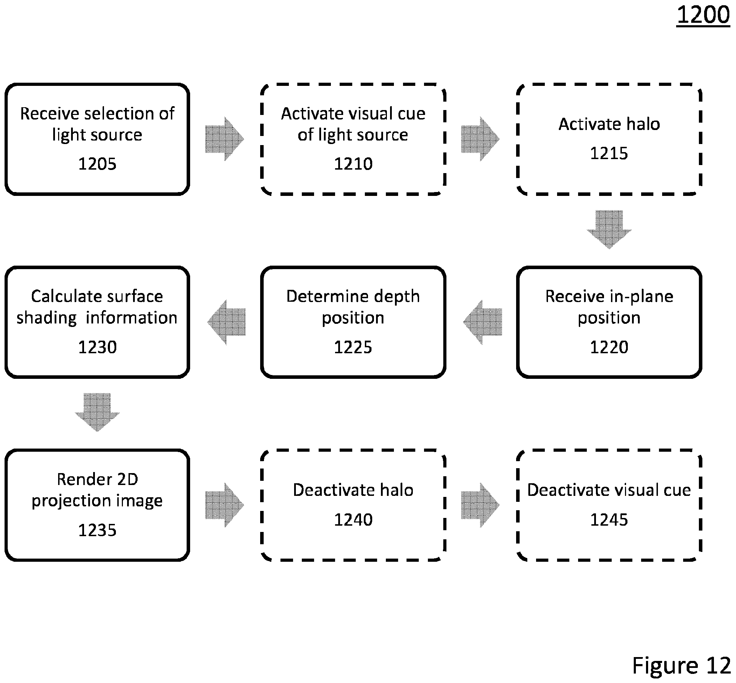

Figure 12 is a flowchart of a method 1200 for positioning a simulated light source within a 3D data set for rendering 2D projections from a perspective of a virtual observer of the 3D data set according to an embodiment of the disclosure. In some embodiments, method 1200 may be implemented using the image rendering technique 400 illustrated in Figure 4 and the ultrasound imaging system shown in Figure 3. In some embodiments, a user may select a position of a simulated light source in a 3D data set prior to rendering of a 2D projection image of the 3D data set. In some embodiments, an imaging system may render a 2D projection image from a 3D data set with an initial default light source in a default position. The default light source and position may be pre-programmed into the imaging system and/or may be set by a user. In some embodiments, the default light source may be an external directional light source at fixed distance from the data set. In some embodiments, the default light source may be a multidirectional light source positioned within or near the 3D data set. At Step 1205, an imaging system may receive a selection of a simulated light source for rendering a 2D projection image of a 3D data set. In some embodiments, a user may select a simulated light source. The user may select the light source via a user interface such as user interface 24 in Figure 1 or user interface 810 in Figure 8. In some embodiments, the user may navigate through a user interface to enter a lighting control mode of the imaging system. In some embodiments, the user may tap a button or a touch screen to select the light source. Optionally, the user and/or imaging system may activate a visual cue of the light source at Step 1210. That is, the user may choose to have the light source rendered in the image as an object (e.g., an orb). In some embodiments, the light source may be rendered in the image by default. Optionally, the user and/or imaging system may activate a halo around the light source at Step 1215. In some embodiments, the light source may be rendered with a halo by default. In some embodiments, the user may prefer to render the image without the halo.

At Step 1220, the imaging system may receive an indication, responsive to user input, of an in-plane position of the simulated light source in a plane corresponding to a projection plane of the 2D projection image (e.g., image plane 420 of Figure 4). The user may select an in-plane position for the light source. The in-plane position may correspond to a position in the image plane in some embodiments. At Step 1225, a depth position of the simulated light source on an axis normal to the projection plane (e.g., Z-axis) may be determined. According to the present invention, the user selects a depth position for the light source. The depth position may correspond to the depth within the 3D data set in relation to the image plane. In some embodiments, Step 1225 and Step 1220 may be performed in reverse order. In some embodiments, Step 1220 and 1225 may be performed simultaneously. The user may select the in-plane position and depth position by using a track ball, a touch screen, and/or another method and/or user interface such as those described above in reference to Figure 8. The imaging system may then calculate surface shading information for one or more surfaces in the 3D data set based on the in-plane and depth positions at Step 1230. At Step 1235, the imaging system may render the 2D projection image including the shading information on a display. In some embodiments, the imaging system may re-render the image as the position of the light source is moved by the user. That is, the light and shadows of the image may dynamically change as the position of the light source is altered (e.g., the surface shading information may be recalculated). This may allow the user to quickly compare potential positions of the light source and/or investigate features of the image by illuminating portions of the image in sequence. For example, the user may move the light source along a spinal column to examine each vertebra.

Once the light source is in position, the halo, if rendered, may be deactivated at Step 1240. In some embodiments, the user may choose to deactivate it (e.g., via a user interface). In some embodiments, the imaging system may automatically stop rendering the halo when the light source is stationary for a period of time. Alternatively, the halo may continue to be rendered. This may be desirable when the user has chosen a position for the light source that is outside the field of view. Optionally, at Step 1245, the visual cue for the light source may be deactivated. That is, the object rendered as the light source in the image may be removed from the image. The imaging system may deactivate the visual cue for the light source automatically or the user may choose to deactivate the visual cue for the light source. Deactivating the visual cue for the light source may be advantageous when the user wishes to observe minute features illuminated in the image near the light source.

Method 1200 may be performed during image acquisition in some embodiments. For example, the imaging system may render images from a 3D data set acquired from a matrix array ultrasound transducer during an ultrasound exam. Method 1200 may be performed on a 3D data set stored on an imaging system or other computing device (e.g., computer, hospital mainframe, cloud service). For example, a radiologist may review images rendered from a 3D data set acquired during a prior exam.

Although method 1200 is described with reference to a single light source, all or portions of method 1200 are performed and/or repeated for multiple light sources. A user sets a first light source at a shallow depth (e.g., near the image plane), which may provide general lighting to the render volume in the image. The user sets a second light source at a deeper depth. The user may set the second light source close to a region of interest. This may allow the user to highlight features of the region of interest while providing visibility to the features surrounding the region of interest.

As described herein, a simulated light source that may be placed anywhere within and/or surrounding a 3D data set may provide additional illumination options for images rendered from the 3D data set. The simulated light source may be a multidirectional light source in some embodiments. These additional options may allow for rendering of images that are less prone to self-shadowing by other anatomical features and better definition of surfaces and/or thicknesses of tissues.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the scope of the present system as set forth in the claims that follow. Accordingly, the specification and drawings are to be regarded in an illustrative manner and are not intended to limit the scope of the appended claims.

## Claims

1. An ultrasound imaging system (10;800) comprising:
an ultrasound probe (12) configured to receive ultrasound echoes from a subject to image a volume of the subject;
a scan converter (30) configured to generate a three dimensional (3D) data set from the ultrasound echoes;
a volume renderer (34) configured to calculate surface shading information of a surface of the 3D data set (430) based, at least in part, on a location of a simulated light source (405;405a-e;705;915a-c;1015;1115) relative to the 3D data set (430) and render a two dimensional (2D) projection image of the 3D data set (430), the 2D projection image including the shading information; and
a user interface (24;805) comprising:
a display (38;810;905;1005) configured to display the 2D projection image; and
an input device comprising a user input element configured to receive user input to position the simulated light source (405;405a-e;705;915a-c;1015;1115) at a location within the 3D data set (430), wherein the simulated light source (405;405a-e;705;915a-c;1015;1115) comprises a plurality of simulated light sources including a first light source at a shallow depth near the image plane and a second light source at a deeper depth within the 3D data set (430) relative to the first light source.

2. The imaging system (10;800) of claim 1, wherein the simulated light source (405;405a-e;705;915a-c;1015;1115) is a multidirectional light source.

3. The imaging system (10;800) of claim 1, wherein the surface represents a boundary between two different materials of the imaged volume.

4. The imaging system (10;800) of claim 1, wherein the volume renderer (34) is configured to calculate first shading information responsive to an indication that the simulated light source (405;405a-e;705;915a-c;1015;1115) is located at a given distance in front of the surface as perceived by a virtual observer and calculate second shading information different from the first shading information responsive to an indication that the simulated light source (405;405a-e;705;915a-c;1015;1115) is located within the 3D data set (430) at the given distance behind the surface as perceived by the virtual observer.

5. The imaging system (800) of claim 1, wherein the user interface (805) comprises a trackball (825), a touchpad, a touch screen (815), or a combination thereof, and wherein the user interface element is provided via the trackball (825), the touchpad, or the touch screen (815) .

6. The imaging system (800) of claim 1, wherein the user input element comprises a GUI displayed on a touch screen (815) of the ultrasound system (800), and wherein the GUI comprises a visual cue of the simulated light source (405;405a-e;705;915a-c;1015;1115) displayed in the 2D projection image along with the rendered 3D data set (430), and wherein the visual cue is movable, responsive to user input, to allow the user to dynamically change the location of the simulated light source (405;405a-e;705;915a-c;1015;1115) in relation to the rendered 3D data set (430).

7. The imaging system (10;800) of claim 1, wherein the volume renderer (34) is configured to render a visual cue of the simulated light source (405;405a-e;705;915a-c;1015;1115) in the 2D projection image.

8. The imaging system (10;800) of claim 7, wherein the visual cue comprises an orb.

9. The imaging system (10;800) of claim 7, wherein a size of the visual cue is based, at least in part, on a depth of the simulated light source (405;405a-e;705;915a-c;1015;1115) within the 3D data set (430) .

10. The imaging system (10;800) of claim 1, wherein the user input element is a first user input element, and wherein the input device comprises a second user input element configured to receive user input to set an intensity of the simulated light source (405;405a-e;705;915a-c;1015;1115) .

11. A computer implemented method comprising:
receiving (1205) a selection of a simulated light source (405;405a-e;705;915a-c;1015;1115) for rendering a 2D projection image of a 3D data set (430), wherein the 3D data set (430) is constructed from ultrasound echoes received from a volume of a subject;
receiving (1220) an indication, responsive to user input, of an in-plane position of the simulated light source (405;405a-e;705;915a-c;1015;1115) in a plane corresponding to a projection plane of the 2D projection image;
selecting (1225) a depth position of the simulated light source (405;405a-e;705;915a-c;1015;1115) within the 3D data set (430) on an axis normal to the projection plane, wherein the simulated light source (405;405a-e;705;915a-c;1015;1115) comprises a plurality of simulated light sources including a first light source at a shallow depth near the image plane and a second light source at a deeper depth within the 3D data set (430) relative to the first light source;
calculating (1230) surface shading information of a surface of the 3D data set (430) based, at least in part, on the in-plane and depth positions; and
rendering (235) the 2D projection image including the shading information on a display (38;810;905;1005).

12. The method of claim 11, further comprising after receiving (1205) the selection of the simulated light source (405;405a-e;705;915a-c;1015;1115), activating (1210) a visual cue of the simulated light source (405;405a-e;705;915a-c;1015;1115) in the rendered 2D projection image.

13. The method of claim 12, wherein the visual cue comprises a halo (1020;1120) .

14. The method of claim 13, further comprising deactivating (1240,1245) the visual cue and halo after the in-plane position has been received and the depth position has been selected.

## Patentansprüche

1. Ein Ultraschallbildgebungssystem (10; 800), das Folgendes umfasst:
eine Ultraschallsonde (12), die das Ultraschallecho eines Patienten empfängt, um ein Volumen des Patienten darzustellen;
einen Scan-Konverter (30), der anhand der Ultraschallechos einen dreidimensionalen Datensatz (3D) erstellt;
einen Volumen-Renderer (34), der die Flächenschattierungsdaten einer Fläche des 3D-Datensatzes (430) zumindest teilweise beruhend auf der Position einer simulierten Lichtquelle (405; 405a-e; 705; 915a-c; 1015; 1115) relativ zum 3D-Datensatz (430) berechnet und ein zweidimensionales Projektionsbild (2D) des 3D-Datensatzes (430) darstellt, wobei das 2D-Projektionsbild die Schattierungsdaten umfasst; und eine Benutzerschnittstelle (24; 805), die Folgendes umfasst:
eine Anzeige (38; 810; 905; 1005) zum Anzeigen des 2D-Projektionsbilds; und
ein Eingabegerät mit einem Benutzereingabeelement, das die Benutzereingaben für das Positionieren der simulierten Lichtquelle (405; 405a-e; 705; 915a-c; 1015; 1115) an einer Position im 3D-Datensatz (430) erhält, wobei die simulierte Lichtquelle (405; 405a-e; 705; 915a-c; 1015; 1115) mehrere simulierte Lichtquellen einschließlich einer ersten Lichtquelle in einer geringen Tiefe in der Nähe der Bildebene sowie einer zweiten Lichtquelle in einer relativ zur ersten Lichtquelle größeren Tiefe im 3D-Datensatz (430) umfasst.

2. Das Bildgebungssystem (10; 800) gemäß Anspruch 1, wobei es sich bei der simulierten Lichtquelle (405; 405a-e; 705; 915a-c; 1015; 1115) um eine multidirektionale Lichtquelle handelt.

3. Das Bildgebungssystem (10; 800) gemäß Anspruch 1, wobei die Oberfläche einer Grenze zwischen zwei verschiedenen Materialien des abgebildeten Volumens entspricht.

4. Das Bildgebungssystem (10; 800) gemäß Anspruch 1, wobei der Volumen-Renderer (34) die ersten Schattierungsdaten berechnet, wenn er die Information erhält, dass sich die simulierte Lichtquelle (405; 405a-e; 705; 915a-c; 1015; 1115) aus der Perspektive eines virtuellen Betrachters im festgelegten Abstand vor der Fläche befindet, während die zweiten, sich von den ersten Schattierungsdaten unterscheidenden Schattierungsdaten berechnet werden, wenn die Information erhalten wird, dass sich die simulierte Lichtquelle (405; 405a-e; 705; 915a-c; 1015; 1115) im 3D-Datensatz (430) aus der Perspektive eines virtuellen Betrachters im festgelegten Abstand hinter der Fläche befindet.

5. Das Bildgebungssystem (800) gemäß Anspruch 1, wobei die Benutzerschnittstelle (805) einen Trackball (825), einen Touchpad, einen Touchscreen (815) oder eine entsprechende Kombination umfasst, und wobei das Benutzerschnittstellenelement über den Trackball (825), den Touchpad oder den Touchscreen (815) bereitgestellt wird.

6. Das Bildgebungssystem (800) gemäß Anspruch 1, wobei das Benutzereingabeelement eine grafische Benutzeroberfläche (GUI) umfasst, die auf dem Touchscreen (815) des Ultraschallsystems (800) angezeigt wird, und wobei die GUI einen visuellen Hinweis auf die simulierte Lichtquelle (405; 405a-e; 705; 915a-c; 1015; 1115) bereitstellt, die im 2D-Projektionsbild gemeinsam mit dem dargestellten 3D-Datensatz (430) angezeigt wird, und wobei der visuelle Hinweis mithilfe einer Benutzereingabe verschoben werden kann, um die Position der simulierten Lichtquelle (405; 405a-e; 705; 915a-c; 1015; 1115) relativ zum dargestellten 3D-Datensatz (430) dynamisch zu ändern.

7. Das Bildgebungssystem (10; 800) gemäß Anspruch 1, wobei der Volumen-Renderer (34) einen visuellen Hinweis auf die simulierte Lichtquelle (405; 405a-e; 705; 915a-c; 1015; 1115) im 2D-Projektionsbild anzeigt.

8. Das Bildgebungssystem (10; 800) gemäß Anspruch 7, wobei der visuelle Hinweis eine Kugel umfasst.

9. Das Bildgebungssystem (10; 800) gemäß Anspruch 7, wobei die Größe des visuellen Hinweises zumindest teilweise auf der Tiefe der simulierten Lichtquelle (405; 405a-e; 705; 915a-c; 1015; 1115) im 3D-Datensatz (430) beruht.

10. Das Bildgebungssystem (10; 800) gemäß Anspruch 1, wobei es sich beim Benutzereingabeelement um ein erstes Benutzereingabeelement handelt, und wobei das Eingabegerät ein zweites Benutzereingabeelement umfasst, das anhand der Benutzereingaben die Intensität der simulierten Lichtquelle (405; 405a-e; 705; 915a-c; 1015; 1115) festlegt.

11. Eine auf einem Computer ausgeführte Methode, die folgende Schritte umfasst:
Abrufen (1205) der Auswahl einer simulierten Lichtquelle (405; 405a-e; 705; 915a-c; 1015; 1115) für das Darstellen eines 2D-Projektionsbilds eines 3D-Datensatzes (430), wobei der 3D-Datensatz (430) anhand des von einem Volumen des Patienten abgerufenen Ultraschallechos erstellt wird;
anhand einer Benutzereingabe Abrufen (1220) einer Information über die Ebenenposition der simulierten Lichtquelle (405; 405a-e; 705; 915a-c; 1015; 1115) auf einer Ebene, die der Projektionsebene des 2D-Projektionsbilds entspricht;
Auswählen (1225) einer Tiefenposition der simulierten Lichtquelle (405; 405a-e; 705; 915a-c; 1015; 1115) im 3D-Datensatz (430) auf einer normal zur Projektionsebene verlaufenden Achse, wobei die simulierte Lichtquelle (405; 405a-e; 705; 915a-c; 1015; 1115) mehrere simulierte Lichtquellen einschließlich einer ersten Lichtquelle in einer geringen Tiefe in der Nähe der Bildebene sowie einer zweiten Lichtquelle in einer relativ zur ersten Lichtquelle größeren Tiefe im 3D-Datensatz (430) umfasst.
Berechnen (1230) der Flächenschattierungsdaten des 3D-Datensatzes (430) zumindest teilweise beruhend auf den Ebenen- und Tiefenpositionen; und
Darstellen (235) des 2D-Projektionsbilds einschließlich der Schattierungsdaten auf einer Anzeige (38; 810; 905; 1005).

12. Die Methode gemäß Anspruch 11, wobei zudem im Anschluss an das Abrufen (1205) der Auswahl der simulierten Lichtquelle (405; 405a-e; 705; 915a-c; 1015; 1115) im dargestellten 2D-Projektionsbild ein visueller Hinweis auf die simulierte Lichtquelle (405; 405a-e; 705; 915a-c; 1015; 1115) aktiviert (1210) wird.

13. Die Methode gemäß Anspruch 12, wobei der visuelle Hinweis einen Halo (1020; 1120) umfasst.

14. Die Methode gemäß Anspruch 13, die zudem das Deaktivieren (1240, 1245) des visuellen Hinweises und des Halos umfasst, nachdem die Ebenenposition abgerufen und die Tiefenposition ausgewählt wurde.

## Revendications

1. Un système d'imagerie par ultrasons (10 ;800) comprenant :
une sonde à ultrasons (12) configurée pour recevoir échos ultrasonores d'un sujet pour produire une image de son volume ;
un convertisseur de balayage (30) configuré pour générer un ensemble de données en trois dimensions (3D) à partir d'échos ultrasonores ;
un appareil de rendu de volume (34) configuré pour calculer les informations d'ombrage d'une surface de l'ensemble de données 3D (430) en fonction, au moins partiellement, de l'emplacement d'une source lumineuse simulée (405 ; 405a-e ; 705 ; 915a-c ; 1015 ; 1115) relativement à l'ensemble de données en 3D (430) et rendre une image projetée en deux dimensions (2D) de l'ensemble de données en 3D (430), l'image projetée en 2D incluant les informations d'ombrage ; et
une interface utilisateur (24 ; 805) comprenant :
un écran (38 ; 810 ; 905 ; 1005) configuré pour afficher l'image projetée en 2D et
un dispositif de saisie comprenant un élément de saisie utilisateur configuré pour recevoir la saisie utilisateur, afin de positionner la source lumineuse simulée (405 ; 405a-e ; 705 ; 915a-c ; 1015 ; 1115) à un emplacement au sein de l'ensemble de données 3D (430), la source lumineuse simulée (405 ; 405a-e ; 705 ; 915a-c ; 1015 ; 1115) comprenant plusieurs sources lumineuses simulées, notamment une première source lumineuse à faible profondeur près du plan d'image et une deuxième source lumineuse à une plus grande profondeur au sein de l'ensemble de données 3D (430) que la première source lumineuse.

2. Le système d'imagerie (10 ; 800) de la revendication 1, la source lumineuse simulée (405 ; 405a-e ; 705 ; 915a-c ; 1015 ; 1115) étant multidirectionnelle.

3. Le système d'imagerie (10 ; 800) de la revendication 1, la surface représentant une limite entre les deux différents éléments du volume rendu par imagerie.

4. Le système d'imagerie (10 ; 800) de la revendication 1, l'appareil de rendu de volume (34) étant configuré pour calculer les premières informations d'ombrage répondant à une indication selon laquelle la source lumineuse simulée (405 ; 405a-e ; 705 ; 915a-c ; 1015 ; 1115) est située à une distance donnée devant la surface selon la perception d'un observateur virtuel, et pour calculer un deuxième ensemble d'informations d'ombrage, différent des premières informations d'ombrage répondant à une indication que la source lumineuse simulée (405 ; 405a-e ; 705 ; 915a-c ; 1015 ; 1115) est située dans l'ensemble de données 3D (430) à une distance donnée sous la surface selon la perception d'un observateur virtuel.

5. Le système d'imagerie (800) de la revendication 1, l'interface utilisateur (805) comprenant une boule de commande (825), un pavé tactile, un écran tactile (815), ou une combinaison de ces derniers, et l'élément d'interface utilisateur étant fourni via la boule de commande (825), le pavé tactile ou l'écran tactile (815).

6. Le système d'imagerie (800) de la revendication 1, l'élément de saisie utilisateur comprenant une GUI (interface graphique utilisateur) affichée sur l'écran tactile (815) du système à ultrasons (800) et la GUI comprenant un signal visuel de la source lumineuse simulée (405 ; 405a-e ; 705 ; 915a-c ; 1015 ; 1115) affichée dans l'image projetée en 2D avec l'ensemble de données 3D rendu (430), et le signal visuel pouvant être déplacé, en réponse à la saisie utilisateur, pour permettre à l'utilisateur de changer de façon dynamique l'emplacement de la source lumineuse simulée (405 ; 405a-e ; 705 ; 915a-c ; 1015 ; 1115) par rapport à l'ensemble de données 3D rendu (430) .

7. Le système d'imagerie (10 ; 800) de la revendication 1, l'appareil de rendu de volume (34) étant configuré pour rendre le signal visuel de la source lumineuse simulée (405 ; 405a-e ; 705 ; 915a-c ; 1015 ; 1115) dans l'image projetée en 2D.

8. Le système d'imagerie (10 ; 800) de la revendication 7, le signal visuel comprenant l'orbe.

9. Le système d'imagerie (10 ; 800) de la revendication 7, la taille du signal visuel dépendant, au moins partiellement, de la profondeur de la source lumineuse simulée (405 ; 405a-e ; 705 ; 915a-c ; 1015 ; 1115) dans l'ensemble de données en 3D (430).

10. Le système d'imagerie (10 ; 800) de la réclamation 1, l'élément de saisie utilisateur étant un premier élément de saisie utilisateur, le dispositif de saisie comprenant un deuxième élément de saisie utilisateur configuré pour recevoir la saisie utilisateur afin de régler l'intensité de la source lumineuse simulée (405 ; 405a-e ; 705 ; 915a-c ; 1015 ; 1115) .

11. Une méthode implémentée par ordinateur, comprenant :
réception (1205) de la sélection d'une source lumineuse simulée (405 ; 405a-e ; 705 ; 915a-c ; 1015 ; 1115) pour le rendu d'une image projetée en 2D d'un ensemble de données 3D (430), l'ensemble de données en 3D (430) étant constitué à partir des échos ultrasonores reçus du volume d'un sujet ;
réception (1220) d'une indication, en réponse à la saisie utilisateur, de l'emplacement de la source lumineuse simulée (405 ; 405a-e ; 705 ; 915a-c ; 1015 ; 1115) dans un plan correspondant à un plan de projection de l'image projetée en 2D ;
sélection (1225) d'une profondeur de la source lumineuse simulée (405 ; 405a-e ; 705 ; 915a-c ; 1015 ; 1115) dans l'ensemble de données en 3D (430) sur un axe normal par rapport au plan de projection, la source lumineuse simulée (405 ; 405a-e ; 705 ; 915a-c ; 1015 ; 1115) comprenant plusieurs sources lumineuses simulées, notamment une première source lumineuse à faible profondeur, près du plan d'image,
et une deuxième source lumineuse à une plus grande profondeur dans l'ensemble de données en 3D (430) que la première source lumineuse ;
calcul (1230) des informations d'ombrage d'une surface de l'ensemble de données en 3D (430) en fonction, au moins partiellement, des positions dans le plan et en profondeur ; et
rendu (235) de l'image projetée en 2D incluant les informations d'ombrage à l'écran (38 ; 810 ; 905 ; 1005) .

12. La méthode de la revendication 11, comprenant la réception (1205) de la source lumineuse simulée (405 ; 405a-e ; 705 ; 915a-c ; 1015 ; 1115) sélectionnée, et l'activation (1210) du signal visuel de la source lumineuse simulée (405 ; 405a-e ; 705 ; 915a-c ; 1015 ; 1115) dans l'image projetée en 2D qui est rendue.

13. La méthode de la revendication 12, le signal visuel comprenant un halo (1020 ; 1120).

14. La méthode de réclamation 13, comprenant la désactivation (1240, 1245) du signal visuel et du halo une fois la position dans le plan reçue et la position de profondeur sélectionnée.
